# EUROPÄISCHE PATENTSCHRIFT

(11) **EP 0 427 761 B1**
(45) Veröffentlichungstag und Bekanntmachung des Hinweises auf die Patenterteilung: **05.05.1993**
(21) Anmeldenummer: 89908823.1
(22) Anmeldetag: 02.08.1989
(51) Int. Cl.: A61L 11/00

(54) **BEHEIZTE FÖRDERVORRICHTUNG UND VERFAHREN ZUM DESINFIZIEREN VON KONTAMINIERTEM, ZERKLEINERTEM KRANKENHAUSMÜLL**
PROCESS AND HEATED CONVEYOR FOR DISINFECTING CONTAMINATED, SIZE-REDUCED HOSPITAL REFUSE
PROCEDE ET DISPOSITIF DE TRANSPORT CHAUFFE POUR LA DESINFECTION DE DECHETS CONTAMINES BROYES D'HOPITAUX

(30) Priorität: 08.08.1988 DE 8810087 U
(43) Veröffentlichungstag der Anmeldung: 22.05.1991
(73) Patentinhaber: ROLAND, Rolf Emil, D-66557 Illingen (DE)
(72) Erfinder: ROLAND, Rolf Emil, D-66557 Illingen (DE)
(74) Vertreter: Flaccus, Rolf-Dieter, Dr.
(86) Internationale Anmeldenummer: EP8900904
(87) Internationale Veröffentlichungsnummer: WO9001340

(56) Entgegenhaltungen:
- EP-A- 0 277 507
- AU-B- 5 457 380
- DE-C- 3 505 570
- DE-U- 8 702 583
- FR-A- 2 506 735

## Beschreibung

Die Erfindung betrifft eine beheizte Fördervorrichtung und ein Verfahren zum Desinfizieren von kontaminiertem, zerkleinertem Krankenhausmüll. Eine bekannte Fördervorrichtung dieser Art besteht aus zwei in zwei offenen Halbschalen gegenläufig umlaufenden Förderschnecken, wobei die Wände der Halbschalen durch Thermoöl beheizt sind. Die Doppelschnecken sind innerhalb eines mit einer Eingangsöffnung, einer Ausgangsöffnung und einer Abluftöffnung versehenen Gehäuses angeordnet (Gebrauchsmusterschrift 87 02 583). Die Verwendung von Schnekkenförderern hat sich im Betrieb insofern als ungünstig erwiesen, als die Durchsatzleistung derartiger Desinfektionsanlagen nicht den Erwartungen entspricht. Durch die rotierende Dewegung der Schneckengänge wird das zu desinfizierende Gut zu seitlichen Anhäufungen verschoben, so daß Kontaktflächen der beheizten Wandungen der Halbschalen umwirksam sind. Eine ausreichende Desinfizierung ist nur über eine sehr niedrige Transportgeschwindigkeit zu erhalten, um zu einer ausreichenden Verweilzeit zu gelangen. Ferner wirft die Verwendung von Thermoöl zur Beheizung der Fördervorrichtung Umweltproblem auf, sei es wegen der Altölentsorgung, Leckagen oder der Entstehung von schädlichen Dämpfen bei Überhitzung.

Es ist daher Aufgabe der Erfindung, eine beheizte Fördervorrichtung und ein Verfahren zum Desinfizieren von kontaminiertem, zerkleinerte, Krankenhausmüll bereitzustellen mit verbesserter Desinfektions- und Durchsatzleistung. Diese Aufgabe wird gemäß der Erfindung dadurch gelöst, daß die Vorrichtung aus einem sogenannten Kratzförderer mit zwei endlosen Gliederketten und daran befestigten Mitnehmern besteht, daß der Kratzförderer in einem bis auf eine obere Eingansöffnung, eine Ausgangsöffnung und eine Abluftöffnung geschlossenen Gehäuse angeordnet ist, dessen Bodenwand und langen Seitenwände durch elektrische Widerstandsheizung beheizbar sind, daß durch zwei senkrechte Blechwände ein Einfallschacht gebildet ist, in den mittels Dampfdüsen gespannter und gesättigter Dampf einsprühbar ist und daß die stromabwärts angeordnete Blechwand eine Überlauföffnung hat.

Die Aufgabe wird weiterhin gelöst durch ein Verfahren zur Desinfektion gemäß den Merkmalen der Ansprüche 9 - 15.

Die Verwendung eines in der Fördertechnik bekannten Kratzförderers mit umlaufenden Mitnehmern beseitigt das Problem der Anhäufung des zu desinfizierenden Gutes, durch die beheizte Kontaktflächen bloßgelegt werden könnten. Die Beheizung der Bodenwand sowie der langen Seitenwände des Gehäuses durch elektrische Widerstandsheizung ist nicht nur umweltfreundlich, sondern bietet auch die Möglichkeit der schnellen Regulierung und Abschaltung der Heizenergie. Durch getrennte Steuerung der Heizelemente läßt sich auch in vorteilhafter Weise der Wärmehaushalt innerhalb des Förderers konstant halten.

Indem ein Fallschacht gebildet ist, durch den das eingegebene Gut bis auf die Bodenplatte herabfällt und das in dem Fallschacht befindliche Gut bereits durch gepanntem und gesättigtem Dampf erhitzt und angefeuchtet wird, wird die Desinfizierung bereits im Fallschacht eingeleitet bzw. vorbereitet. Die stromabwärts angeordnete Blechwand des Füllschachtes mit ihrer Überlauföffnung führt dazu, daß ein Teil des in dem Füllschacht befindlichen Gutes, das durch die Mitnehmer des Untertrums der Gliederketten abgestreift und in Förderrichtung bewegt wird, durch die Überlauföffnung unter Umwälzung auf die beheizte Bodenplatte herabfällt.

Bei dem erfindungsgemäßen Verfahren wird zur Desinfektion kondensatfreier gespannter und gesättigter Dampf, vorzugsweise einer Temperatur von 140°C verwendet. Die Temperatur des Bodens wird nach einer Ausführungsform so eingestellt, daß sie unter der Temperatur des eingesetzten gespannten und gesättigten Dampfes liegt, die Temperatur der Seitenwände wird so gewählt, daß sie derjenigen des Heißdampfes mindestens gleich ist oder höher liegt.

Vorzugsweise überschreitet die Temperatur am Boden einen Bereich von 119 bis 125°C nicht. Der Einsatz von 140°C-Dampf ist bevorzugt, die Temperatur der Seitenwände beträgt dabei vorzugsweise 150°C.

Das erfindungsgemäße Verfahren ermöglicht Verweilzeiten des zu desinfizierenden Materials bis zu 15 Minuten. Kürzere Desinfektionszeiten, z.B. 10 Minuten sind - abhängig von der Art der Kontaminierung - erzielbar.

Zum ungestörten Lauf der Gliederketten auf ihren Führungen ist es zweckmäßig, daß die Führungen der Gliederketten durch Abdeckungen vor herabfallendem Krankenhausmüll geschützt sind.

Eine weitere, den Wirkungsgrad der beheizten Fördervorrichtung erhöhende Maßnahme besteht gemäß der Erfindung darin, daß unterhalb der bewegungsbahn der Mitnehmer des Obetrums der Gliederketten ein beheizbares Zwischenblech angeordnet ist, das unterhalb der Eingangsöffnung beginnt und im Bereich des Fallschachtes endet, wobei die Eingangs- und Ausgangsöffnungen annähernd übereinander liegen. Diese Lösung bringt nicht nur eine erwünschte Umwälzung des zu behandelnden Gutes mit sich, sondern auch eine bedeutende Vergrößerung der beheizten Kontaktfläche, über die das zu behandelnde Gut vorgeschoben wird, und zwar bei vergleichsweise gleicher Länge des Kratzförderers. Über den Zwischenboden können weitere Dampfdüsen vorgesehen werden.

Da Krankenhausmüll in großem Umfang Gegenstände aus Kunststoff enthält, der bei der Erhitzung gasförmige Schadstoffe freisetzt, ist zwecks größtmöglicher Dichtheit des Gehäuses, das mit einer Abluftöffnung mit Filter versehen ist, die der Ausgangsöffnung nächstgelegene angetriebene Achse der Umlenkräder der Gliederketten außerhalb des Gehäuses gelagert und gegenüber den Seitenwänden mittels Stopfbüchsen abgedichtet, wogegen die andere Achse von Umlenkrädern stirnseitig an den Seitenwänden des Gehäuses fixiert ist und deren Umlenkräder auf der Achse gelagert sind.

Die Mitnehmer der Gliederketten sind zweckmäßig mit temperaturbeständigen Gummi- oder Kunststoff-Belägen versehen, da die Mitnehmer während ihrer Bewegung über die Bodenwand und gegebenenfalls das Zwischenblech schleifen.

Schließlich ist es geboten, zur Vermeidung von Wärmeverlusten das Gehäuse mit einer Einhausung zur Aufnahme einer Wärmedämmung zu umgeben, wobei die Einhausung mit den Eingangs- und Ausgangsöffnungen sowie der Abluftöffnung versehen ist. Das Deckelteil der Einhausung ist zweckmäßig mit einem Schnellverschluß versehen, um Störungen schnell beseitigen zu können.

In der Zeichnung ist ein Ausführungsbeispiel des Gegenstandes der Erfindung dargestellt, und zwar zeigen
- Fig. 1: einen senkrechten Querschnitt in schematischer Darstellung mit einer wärmedämmenden Einhausung, und
- Fig. 2: einen Schnitt nach der Linie II-II in Fig. 1 ohne die Einhausung.

Zunächst sei der Kratzförderer beschrieben, der zwei endlose Gliederketten 1, 2 umfaßt, die über Umlenkräder 3, 4 laufen und in Abständen voneinander angeordnete Mitnehmer 6 tragen, die mit Belägen 6a aus temperaturbeständigem Gummi versehen sind. Die Umlenkräder 3, 4 sind auf der nicht angetriebenen Achse 5 drehbar gelagert, die stirnseitig an den langen Seitenwänden 7 eines Gehäuses durch Schrauben fixiert ist, das außer den kurzen Seitenwänden 8 eine Bodenwand 9 und einen Deckel 10 aufweist. Der Antrieb des Kratzförderers erfolgt über eine Achse 11, die Außerhalb des Gehäuses in Lagerböcken 12 gelagert ist, die auf einer an den Seitenwänden 7 fixierten Konsole 13 abgestützt sind.

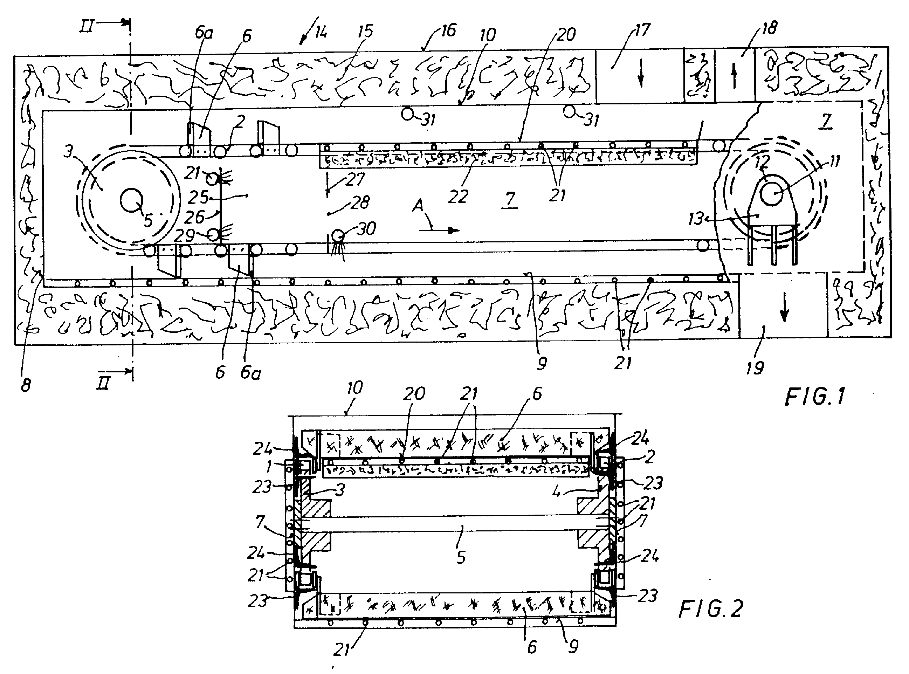

Das aus den Seitenwänden 7, 8, der Bodenwand 9 und dem Deckel 10 gebildete Gehäuse ist von einer Einhausung 14 umgeben, die eine Wärmedämmung 15 aus aluminium-kaschierter Mineralwolle in sich aufnimmt. Der Deckel 16 der Einhausung 14 ist durch einen nicht dargestellten Schnellverschluß leicht abnehmbar. Der Deckelteil 16 der Einhausung 14 hat eine Eingangsöffnung 17 für den einzufüllenden zerkleinerten Krankenhausmüll, eine Gasabzugsöffnung 18, an die ein nicht dargestelltes Rohr mit Filter angeschlossen ist, und eine Ausgangsöffnung 19. Die Eingangsöffnung 17 und die Ausgangsöffnung 19 liegen bei der in Fig. 1 dargestellten beheizbaren Fördervorrichtung annähernd übereinander. Es versteht sich, daß allen Öffnungen in der Einhausung 14 entsprechende Öffnungen im Deckel 10 und in der Bodenwand 9 des Gehäuses zugeordnet sind.

Unterhalb der Eingangsöffnung 17 beginnt ein Zwischenboden 20 in Gestalt eines flachen Hohlkastens, in den Heizstäbe 21 einer Widerstandsheizung eingebaut sind. Unterhalb der Heizstäbe 21 enthält der Zwischenboden 20 eine Wärmedämmschicht 22. Der Zwischenboden 20 erstreckt sich zwischen den Gliederketten 1, 2 und ist an oberen Winkeln 23 gehaltert (Fig. 2), die an den Seitenwänden 7 des Gehäuses befefestigt sind und zur Führung der Gliederketten 1, 2 dienen. Über ebensolche Winkel 24 sind die Gliederketten von oben abgedeckt.

Der Zwischenboden 20 erstreckt sich bis in den Bereich eines Einfüllschachtes 25, der von senkrechten Blechwänden 26, 27 gebildet ist. Die in Pfeilrichtung A stromabwärts angeordnete Seitenwand 27 hat eine Überlauföffnung 28 in der Nähe des Untertrums der Gliederketten 1, 2. Hinter der Blechwand 26 befinden sich Rohre 29, die an eine nicht dargestellte Dampferzeugungsanlage angeschlossen sind und mit Dampfdüsen versehen sind, die den Heißdampf in den Fallschacht 25 sprühen. Ein weiteres Heißdampf führendes Rohr 30 ist neben der Blechwand 27 vorgesehen, um das auf der Bodenwand 9 in Pfeilrichtung A bewegte Gut zu behandeln. Die Bodenwand 9 und die Seitenwände 7 sind ebenfalls kastenförmig ausgebildet und enthalten Heizstäbe 21. Auch oberhalh des Zwischenbodens 20 sind Dampf führende Rohre 31 mit Dampfdüsen vorgesehen.

Die Wirkungsweise der beschriebenen Vorrichtung ist folgende:

Der zerkleinerte Krankenhausmüll wird über die Eingangsöffnung 17 der Vorrichtung zugeführt und fällt auf den beheizten Zwischenboden 20, auf dem das Gut von den Mitnehmern 6 des Obertrum der Gliederketten 1, 2 des Kratzförderers, der mit Schleichgeschwindigkeit betrieben wird, vorgeschoben wird.

Nach dieser ersten Behandlungsstufe fällt das Gut in den Fallschacht 25 auf die beheizte Bodenwand 9, wo das Gut von den Mitnehmern 6 in Pfeilrichtung A zur Ausgangsöffnung 19 transportiert wird. Im Füllschacht 25 erfolgt eine weitere Aufheizung und Befeuchtung des Gutes durch gespannten und gesättigten Damf aus Dampfdüsen der Rohre 29. Ein Teil des im Fallschacht 25 befindlichen Gutes kann über die Überlauföffnung 28 der Blechwand 27 überlaufen und fällt unter Umwälzung ebenfalls abwärts, wobei über Dampfdüsen des Rohres 30 weiterhin gespannter und gesättigter Dampf eingesprüht wird. Das desinfizierte Gut wird über die Ausgangsöffnung 19 einer nicht dargestellten Preßstation zugeführt.

In einer einfacheren Ausführung der Vorrichtung fehlt der Zwischenboden 20, und die Eingangsöffnung 17 ist über dem Fallschacht 25 angeordnet. Der zerkleinerte Krankenhausmüll gelangt dann durch den Fallschacht unmittelbar auf die beheizte Bodenplatte 9. Es versteht sich, daß durch die Anordnung von Leitblechen insbesondere in dem Fallschacht 25 eine mehrfache Umschichtung des zu behandelnden Gutes erhalten werden kann.

Im übrigen können Vorkehrungen getroffen sein, in dem Gehäuse der Fördervorrichtung einen Dampf-Überdruck einzustellen. Hierzu sind die Abluftöffnung 18 mit einer Überdruckanzeige und die Eingangs- sowie Ausgangsöffnung 17 bzw. 19 mit Schleusen zu versehen.

## Patentansprüche

1. Beheizte Fördervorrichtung zum Desinfizieren von kontaminiertem, zerkleinerten Krankenhausmüll, **dadurch gekennzeichnet**, daß die Vorrichtung aus einem sogenannten Kratzförderer mit zwei endlosen Gliederketten (1, 2) und daran befestigten Mitnehmern (6) besteht, daß der Kratzförderer in einem bis auf eine obere Eingangsöffnung (17), eine Ausgangsöffnung (19) und eine Abluftöffnung (18) geschlossenen Gehäuse angeordnet ist, dessen Bodenwand (9) und langen Seitenwände (8) durch elektrische Widerstansheizung beheizbar sind, daß durch zwei senkrechte Blechwände (26, 27) ein Fallschacht (25) gebildet ist, in den mittels Dampfdüsen gespannter und gesättigter Dampf einsprühbar ist, und daß die stromabwärts angeordnete Blechwand (27) eine Überlauföffnung (28) hat.

2. Fördervorrichtung nach Anspruch 1, dadurch gekennzeichnet, daß die Führungen (23) der Gliederketten (1, 2) durch Abdeckungen (24) geschützt sind.

3. Fördervorrichtung nach Anspruch 1 und 2, dadurch gekennzeichnet, daß unterhalb der Bewegungsbahn der Mitnehmer (6) des Obertrums der Gliederketten (1, 2) ein beheizbarer Zwischenboden (20) angeordnet ist, der unterhalb der Eingangsöffnung (17) beginnt und im Bereich des Fallschachtes (25) endet, wobei die Eingangs- und Ausgangsöffnungen (17, 19) annähernd übereinander liegen.

4. Fördervorrichtung nach Anspruch 3, dadurch gekennzeichnet, daß über dem beheizbaren Zwischenboden (20) weitere Dampfdüsen vorgesehen sind.

5. Fördervorrichtung nach Anspruch 1, dadurch gekennzeichnet, daß die der Ausgangsöffnung nächstgelegene Antriebsachse (11) der Umlenkräder (3, 4) der Gliederketten (1,2) außerhalb des Gehäuses gelagert und gegenüber dessen Seitenwänden (8) mittels Stopfbüchsen abgedichtet sind, wogegen die andere Achse (5) von Umlenkrädern (3, 4) stirnseitig an den Seitenwänden (8) fixiert ist und deren Umlenkräder auf der Achse gelagert sind.

6. Fördervorrichtung nach Anspruch 1 bis 5, dadurch gekennzeichnet, daß die Mitnehmer (6) mit temperaturbeständigen Gummi- oder Kunststoff-Belägen (6a) versehen sind.

7. Fördervorrichtung nach mindestens einem der Ansprüche 1 bis 6, dadurch gekennzeichnet, daß das Gehäuse von einer Einhausung (14) zur Aufnahme einer Wärmedämmung (15) umgeben ist, und daß die Einhausung mit den Eingangs- und Ausgangsöffnungen (17, 19) sowie der Abluftöffnung (18) versehen ist.

8. Fördervorrichtung nach Anspruch 7, dadurch gekennzeichnet, daß die Einhausung (14) ein mit einem Schnellverschluß versehenes Deckelteil (16) hat.

9. Verfahren zum Desinfizieren von kontaminiertem, zerkleinertem Krankenhausmüll, dadurch gekennzeichnet, daß der Müll innerhalb einer beheizten Fördervorrichtung gemäß einem der Ansprüche 1 bis 8 mittels gespanntem und gesättigtem Dampfes desinfiziert wird.

10. Verfahren nach Anspruch 9, dadurch gekennzeichnet, daß zur Desinfektion kondensatfreier Dampf verwendet wird.

11. Verfahren nach Anspruch 9 oder 10, dadurch gekennzeichnet, daß die elektrischen Heizelemente getrennt gesteuert werden.

12. Verfahren nach einem der Ansprüche 9 bis 11, dadurch gekennzeichnet, daß die Temperatur am Boden unter der Temperatur, die Temperatur der Seitenwände über der Temperatur des eingesetzten gespannten und gesättigten Dampfes gehalten wird oder ihr mindestens gleich ist.

13. Verfahren nach Anspruch 12, dadurch gekennzeichnet, daß bei Einsatz von gespanntem und gesättigtem Dampf einer Temperatur von 140°C die Temperatur der Seitenwände auf 150°C gehalten wird.

14. Verfahren nach einem der Ansprüche 9 bis 13, dadurch gekennzeichnet, daß die Temperatur am Boden einen Bereich von 119 bis 125°C nicht überschreitet.

15. Verfahren nach einem der Ansprüche 9 bis 14, dadurch gekennzeichnet, daß die Verweilzeit des zu desinfizierenden Materials in der Vorrichtung bis zu 15 Minuten beträgt.

## Claims

1. Heated conveyor for disinfecting contaminated, size- reduced hospital refuse, characterised in that the device comprises a so-called scraping conveyor with two endless link chains (1, 2), to which flight attachments (6) are secured, that the scraping conveyor is arranged in a housing, whose base (9) and long side walls (8) can be heated by means of electric resistance heating, which is closed with the exception of an upper inlet opening (17), an outlet opening (19) and an air vent (18); that a drop shaft (25) is formed by two vertical sheet metal plates (26, 27), into which compressed, saturated steam can be injected through steam nozzles; and that the sheet metal plate positioned downstream (27) possesses an overflow opening (28).

2. Conveyor according to claim 1, characterised in that the guideways (23) of the link chains (1, 2) are provided with protecting covers (24).

3. Conveyor according to claims 1 and 2, characterised in that a heatable intermediate bottom (20) is positioned below the path of motion of the flight attachments (6) of the upper belt of the link chains (1, 2), which begins below the inlet opening (17) and ends in the area of the drop shaft (25), the inlet and outlet openings (17, 19) lying almost on top of one another.

4. Conveyor according to claim 3, characterised in that further steam nozzles are provided over the heatable intermediate bottom (20).

5. Conveyor according to claim 1, characterised in that the driving axle (11) of the guide wheels (3, 4) of the link chains (1, 2) nearest to the outlet opening is supported outside the housing and sealed vis-a-vis the side walls (8) of the latter by means of glands, the other axle (5) being fixed onto the side walls (8) at the front end by means of guide wheels, the latter in their turn being supported on the axle.

6. Conveyor according to claims 1 to 5, characterised in that the flight attachments (6) are supplied with a temperature-resistant coating of either rubber or plastic (6a).

7. Conveyor according to at least one of the claims 1 to 6, characterised in that the housing is surrounded by an outer casing (14) to incorporate a thermal insulation (15) and that the outer casing is provided with inlet and outlet openings (17, 19) as well as with the air vent (18)

8. Conveyor according to claim 7, characterised in that the outer casing (14) has a cover (16) equipped with a snap closure.

9. Process for disinfecting contaminated size-reduced hospital refuse, characterised in that the refuse is disinfected within a heated conveyor by means of compressed, saturated steam, according to claims 1 to 8.

10. Process according to claim 9, characterised in that steam free of condensate is used in the disinfection.

11. Process according to claims 9 or 10, characterised in that the electrical heating elements are regulated separately.

12. Process according to one of the claims 9 to 11, characterised in that the temperature at the base is kept below the temperature and the temperature of the side walls above or at least equal to the temperature of the compressed, saturated steam employed.

13. Process according to claim 12, characterised in that the temperature of the side walls is kept at 150 degrees Celsius when compressed, saturated steam at a temperature of 140; degrees Celsius is employed.

14. Process according to one of the claims 9 to 13, characterised in that the temperature at the base does not exceed a range between 113 and 125 degrees Celsius.

15. Process according to one of the claims 9 to 14, characterised in that the period of dwell of the material to be disinfected in the device amounts to up to 15 minutes.

## Revendications

1. Dispositif chauffé de transport pour la désinfection de déchets d'hôpitaux contaminés et déchiquetés, caractérisé en ce que le dispositif est constitué de ce que l'on appelle un transporteur racleur avec deux chaînes sans fin (1, 2) à éléments auxquels sont fixés des entraîneurs (6), en ce que le transporteur racleur disposé dans un caisson fermé présentant une ouverture supérieure d'entrée (17), une ouverture de sortie (19) et une ouverture d'évent (18), dont la paroi de fond (9) et les parois latérales allongées (8) peuvent être chauffées par un chauffage à résistance électrique, en ce qu'un puits de chute (25) est formé par deux parois verticales en tôle (26, 27) dans lequel de la vapeur surchauffée et de la vapeur saturée peuvent être injectées au moyen d'injecteurs de vapeur et en ce que la paroi de tôle (27) disposée en aval possède une ouverture de surverse (28).

2. Dispositif de transport selon la revendication 1, caractérisé en ce que les guides (23) des chaînes à éléments (1, 2) sont protégés par des carénages (24).

3. Dispositif de transport selon la revendication 1 et 2, caractérisé en ce qu'en dessous du parcours de déplacement des entraîneurs (6) du brin supérieur des chaînes à éléments (1, 2) est installé un fond intermédiaire chauffé (20) qui commence en dessous de l'ouverture d'entrée (17) et se termine au voisinage du puits de chute (25), les ouvertures d'entrée et de sortie (17, 19) étant situées approximativement l'une au-dessus de l'autre.

4. Dispositif de transport selon la revendication 3, caractérisé en ce que d'autres injecteurs de vapeur sont prévus au-dessus du fond intermédiaire (20) pouvant être chauffé.

5. Dispositif de transport selon la revendication 1, caractérisé en ce que l'axe d'entraînement des roues de renvoi (3, 4) des chaînes à éléments (1, 2) situé le plus près de l'ouverture de sortie est maintenu à rotation à l'extérieur du caisson et est rendu hermétique par rapport aux parois latérales (8) du caisson à l'aide de douilles à bouchon, tandis que l'autre axe (5) des roues de renvoi (3, 4) est fixé à ses extrémités frontales aux parois latérales (8), ces roues de renvoi étant soutenues à rotation sur l'axe.

6. Dispositif de transport selon les revendications 1 à 5, caractérisé en ce que les entraîneurs (6) sont équipés de recouvrement en matière synthétique ou en caoutchouc (6a) résistant à la température.

7. Dispositif de transport selon au moins une des revendications 1 à 6, caractérisé en ce que le caisson est entouré d'un caisson extérieur (14) servant à reprendre une isolation thermique (15) et en ce que le caisson extérieur est doté des ouvertures d'entrée et de sortie (17, 19) ainsi que de l'ouverture d'évent (18).

8. Dispositif de transport selon la revendication 7, caractérisé en ce que le caisson extérieur (14) possède un couvercle (16) doté d'une fermeture rapide.

9. Procédé de désinfection de déchets d'hôpitaux contaminés et déchiquetés, caractérisé en ce que les déchets sont désinfectés dans un dispositif de transport chauffé selon l'une des revendications 1 à 8 à l'aide de vapeur saturée sous pression.

10. Procédé selon la revendication 9, caractérisé en ce que l'on utilise pour la désinfection de la vapeur sèche.

11. Procédé selon la revendication 9 ou 10, caractérisé en ce que les éléments électriques chauffants sont commandés distinctement.

12. Procédé selon l'une des revendications 9 à 11, caractérisé en ce que la température du fond est maintenue en dessous de la température de la vapeur saturée sous pression tandis que la température des parois latérales est maintenue au-dessus de la température de la vapeur saturée sous pression ou au moins lui est égale.

13. Procédé selon la revendication 12, caractérisé en ce que lorsque l'on utilise de la vapeur saturée sous pression à une température de 140° C on maintient la température des parois latérales à 150° C.

14. Procédé selon l'une des revendications 9 à 13, caractérisé en ce que la température du fond ne quitte pas une plage de 119 à 125 ° C.

15. Procédé selon l'une des revendications 9 à 14, caractérisé en ce que le temps de séjour de la matière à désinfecter dans le dispositif vaut jusqu'à 15 minutes.
